# FASCICULE DE BREVET EUROPEEN

(11) **EP 2 266 504 B1**
(45) Date de publication et mention de la délivrance du brevet: **29.03.2017**
(21) Numéro de dépôt: 10184842.2
(22) Date de dépôt: 02.08.2002
(51) Int. Cl.: A61F 2/24, A61F 2/06

(54) **Dispositif et méthode d'implantation d'une endoprothèse**
Verfahren und Vorrichtung zum Einsetzen von Endoprothesen
Device and method to implant an endoprosthesis

(30) Priorité: 03.08.2001 FR 0110444
(43) Date de publication de la demande: 29.12.2010
(62) Demande divisionnaire de: 02291953.4
(73) Titulaire: JenaValve Technology Inc., Wilmington, DE 19801 (US); Medtronic Vascular Galway Limited, Galway (IE)
(72) Inventeur: Bonhoeffer, Philipp, London, Greater London NW19XP (GB); Boudjemline, Younès, 92140, Clamart (FR)
(74) Mandataire: den Braber, Gerard Paul

(56) Documents cités:
- WO-A-00/69367
- WO-A-99/42058
- WO-A1-01/35870
- WO-A1-99/33414
- WO-A2-01/49213
- US-A- 5 053 008
- DATABASE MEDLINE [en ligne] US NATIONAL LIBRARY OF MEDICINE (NLM), BETHESDA, MD, US 2015 PIECHOTA-POLANCZYK ALEKSANDRA ET AL: 'The Abdominal Aortic Aneurysm and Intraluminal Thrombus: Current Concepts of Development and Treatment.' Database accession no. NLM26664891 & FRONTIERS IN CARDIOVASCULAR MEDICINE 2015, vol. 2, 2015, page 19, ISSN: 2297-055X

## Description

### Domaine technique

La présente invention se rapporte à un dispositif d'implantation d'un implant.

Le problème à l'origine de l'invention concernait l'implantation de valves cardiaques. Encore récemment, cela nécessitait une intervention chirurgicale à coeur ouvert, avec des étapes telles que l'arrêt du coeur, la mise en oeuvre d'une circulation sanguine extra-corporelle et le redémarrage du coeur après l'implantation de valves cardiaques de remplacement. Ces opérations chirurgicales sont lourdes et délicates et elles présentent des risques mortels liés aux chocs opératoires.

### Art antérieur

Le document US 5 824 063 décrit ainsi un dispositif portant des valves cardiaques de remplacement, dispositif comprenant un implant tubulaire en matériau synthétique portant intérieurement une valve de remplacement en matériau naturel.

Le document WO 99/42058 décrit un dispositif pour positionner de façon permanente un implant tubulaire dans une artère de façon à ce que l'implant est scellé par rapport à la paroi de l'artère. Le dispositif comprend des éléments pouvant être déployés radialement vers l'extérieur.

Les documents US 5 855 601 et US 5 868 783 décrivent de nouvelles méthodes d'implantation de valves cardiaques, qui présentent l'avantage d'éviter une chirurgie à coeur ouvert. Ces méthodes prévoient l'implantation, par cheminement dans le système de circulation sanguine, d'un dispositif à valve de remplacement cardiaque comprenant un cylindre intravasculaire expansible radialement portant intérieurement une valve biologique. Une partie gonflable d'un cathéter ballon est placée à l'intérieur du cylindre porteur et l'implantation est réalisée par introduction dans une veine et translation jusqu'à la valve défaillante au moyen d'un cathéter. Une visualisation sur écran d'imagerie bidimensionnelle permet de détecter que le cylindre porteur a atteint la position voulue et le cylindre est alors dilaté par gonflage du ballonnet à travers le cathéter et il conserve sa forme expansée. Le ballonnet est alors dégonflé et retiré avec le cathéter. Le cylindre porteur présente une enveloppe étanche qui est ainsi plaquée contre la paroi de l'artère, afin d'éviter un contournement de la valve de remplacement par le flux sanguin.

Toutefois, lorsqu'il s'agit de l'aorte, ce procédé n'est pas applicable car les artères coronaires débouchent à proximité des valves natives défaillantes, si bien que le cylindre porteur est susceptible de les obturer, provoquant la mort du patient.

Le document WO 01/35870 décrit un dispositif de remplacement par voie percutanée d'une valve cardiaque. Deux séries de lames allongées sont disposées autour de la circonférence d'un élément allongé. Les lames peuvent être déployées en corolle de telle sorte que leurs bords tranchants soient placés dans le prolongement les uns des autres. Les lames peuvent être rapprochées de telle sorte que leurs bords circulaires tranchants viennent sectionner la valve native de manière à la séparer du conduit corporel.

Le document WO 01/35870 décrit un système pour l'insertion d'une prothèse de valve cardiaque. Le système comprend un système de valve pliable à base de tissu, un système de transport de valve à base de cathéter, une plate-forme chirurgicale, et un système de repérage et de visualisation de l'appareil.

Le document WO 01/49213 décrit des valvules cardiaques et veineuses prothétiques, un cathéter unique et des techniques minimalement invasives de valvuloplastie percutanée et transluminale et d'implantation des valvules prothétiques.

### But de l'invention

Les inventeurs de la présente demande ont alors pensé à ménager deux ouvertures correspondantes dans la paroi de l'enveloppe du cylindre porteur. Cependant, pour que ces deux ouvertures soient placées en face des deux coronaires, il faut totalement maîtriser le positionnement du cylindre porteur dans l'aorte. Un suivi sur l'écran permettrait bien de vérifier l'avance, ou positionnement axial, du cylindre porteur, mais sa position angulaire ne serait ni visible, ni maîtrisée.

Les demandeurs ont alors trouvé une solution, exposée plus loin, permettant de maîtriser le positionnement du cylindre porteur.

Ils ont alors songé à l'appliquer à la résolution du problème plus général de positionnement d'un dispositif ou véhicule de transport d'un implant dans un élément tubulaire difficile d'accès et pour lequel une imagerie est insuffisante ou même impossible. Le champ d'application peut ainsi concerner des domaines autres que le médical, par exemple l'industrie pétrolière ou nucléaire, pour implanter des capteurs, vannes ou autres.

### Résumé de l'invention

La revendication 1 définit un dispositif conforme à l'invention pour implanter une prothèse de valve. La revendication 7 définit un système conforme à l'invention. Les autres revendications définissent des caractéristiques supplémentaires qui peuvent être avantageusement appliquées pour mettre en oeuvre l'invention.

Grâce à l'invention, on peut faire avancer de façon aveugle le dispositif et ses moyens palpeurs permettent de détecter automatiquement la cavité et de s'y positionner.

On peut ainsi accéder à la position finale voulue, même à travers un rétrécissement de l'élément tubulaire, par exemple une artère d'accès à une artère de plus grand diamètre.

### Brève description des dessins

Les caractéristiques et avantages de la présente invention apparaîtront plus clairement à l'aide de la description suivante d'une forme particulière de réalisation du dispositif de l'invention et d'une variante, ainsi que du procédé de mise en oeuvre de celui-ci, en référence au dessin annexé, dans lequel :
- la figure 1 est une vue en section latérale du dispositif de l'invention, représentant des éléments palpeurs de positionnement et d'ancrage, associés à un cylindre porteur d'une prothèse de valve, le tout recouvert de deux gaines d'activation amovibles concentriques,
- la figure 2 correspond à la figure 1, les éléments palpeurs de positionnement et d'ancrage ayant été déployés radialement par recul axial de la gaine externe,
- la figure 3 correspond aux figures 1 et 2, le cylindre porteur entouré des éléments palpeurs de positionnement et d'ancrage ayant été déployé radialement après recul axial de la gaine interne,
- la figure 4 est une vue latérale du cylindre porteur et des éléments palpeurs de positionnement et d'ancrage,
- la figure 5 est une vue latérale en perspective des éléments palpeurs de positionnement et d'ancrage,
- la figure 6 est une vue de face schématique du dispositif de l'invention, et
- la figure 7 est une coupe latérale schématique de la variante.

### Description détaillée

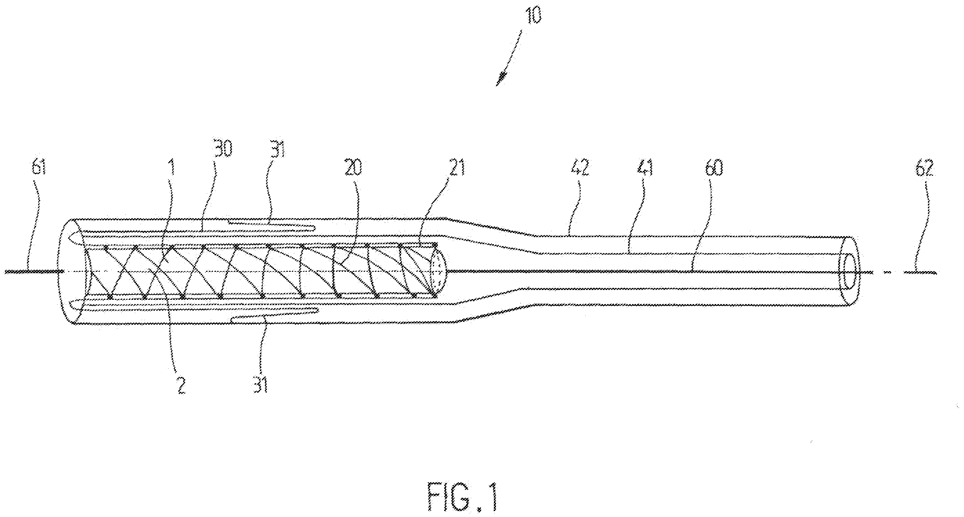

Comme le montre la figure 1, le présent exemple de réalisation correspond au problème médical exposé au début, d'implantation d'une valve de remplacement fonctionnel de la valve native aortique. Le dispositif 10 d'implantation de valves comporte un élément porteur 20 de réception d'implant, solidaire d'une pluralité d'éléments ou doigts palpeurs 30, 31, ici régulièrement répartis angulairement tout autour, de positionnement et d'ancrage par rapport à un relief, précisément une cavité de la paroi aortique, le dispositif 10 étant relié de façon amovible à un cathéter 60 de mise en place. Le dispositif 10 est associé à deux gaines concentriques 41, 42 de commande de téléactivations successives, par expansion radiale, des éléments palpeurs 30, 31 puis de l'élément porteur 20. La direction d'avance du dispositif 10 est donc vers la gauche sur les figures 1 à 3. La référence 62 représente un axe de symétrie et de direction d'entraînement du dispositif 10 et du cathéter 60.

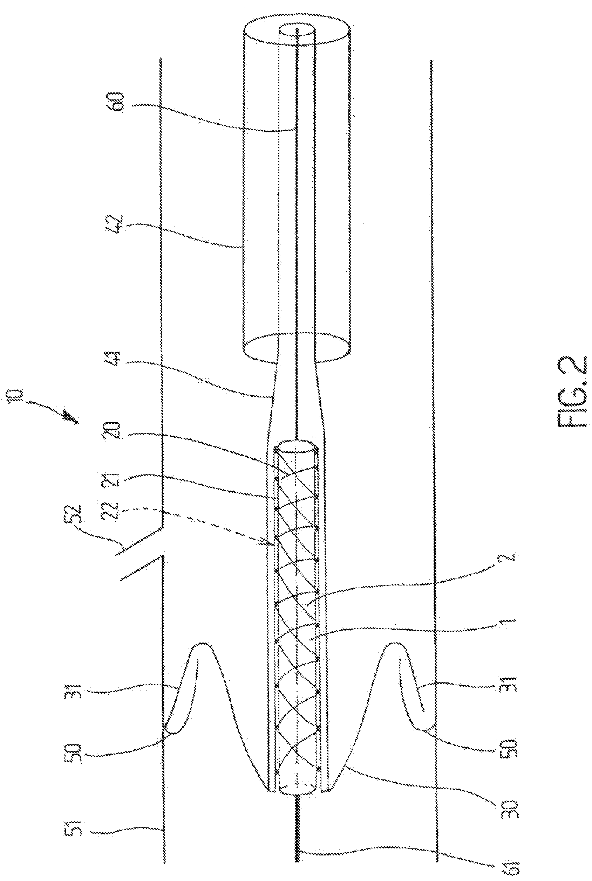

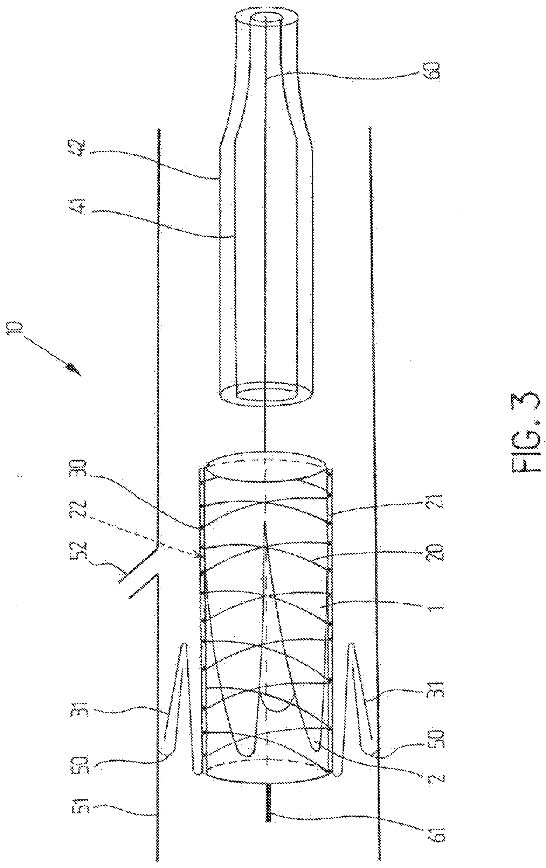

La valve d'implantation forme une prothèse 1 constituée de valvules 2 de la valve dont la forme et la taille correspondent parfaitement, en position fonctionnelle, à celles de valves natives aortiques 50 (fig. 2). La prothèse 1 est fixée à l'élément véhicule porteur 20 de réception d'implant, ici constitué par un treillis cylindrique en un matériau bio-compatible tel que l'acier, des alliages d'or et, de préférence comme ici, le nitinol, constitué d'un alliage nickel-titane à mémoire de forme présentant la faculté de reprendre sa forme après avoir été initialement déformé, ici par compression radiale. La fixation de la prothèse 1 au treillis cylindrique 20 en nitinol est effectuée en des endroits bien définis laissant libres des régions, correspondant aux valvules 2 après leur déploiement tel qu'illustré plus loin en regard de la figure 3, à partir de la position reployée de la figure 2.

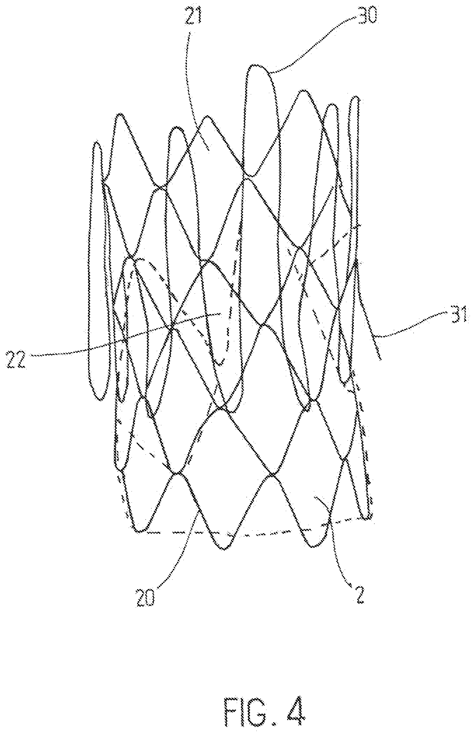

La figure 4 représente le treillis cylindrique 20 dans sa forme déployée, portant intérieurement les valvules 2 aussi déployées, sur lequel sont raccordés les éléments palpeurs 30, 31, ici sous la forme d'une couronne extérieure globalement cylindrique de boucles de fil dont l'une (31) au moins, ici en fait trois, fait saillie latéralement et vers l'avant, à l'opposé du cathéter 60. Dans cet exemple, les boucles 31 s'étendent, en position déployée, dans une direction inclinée d'environ 30 degrés vers l'avant (direction d'avance vers la position visée) par rapport à l'axe 62 du treillis 20 et de la couronne 30. Les éléments palpeurs 30, 31 sont solidarisés au treillis cylindrique 20 de telle manière que leurs positions axiales et angulaires par rapport à celui-ci sont parfaitement définies. L'ensemble, treillis cylindrique 20 et éléments palpeurs 30, 31, est ici constitué en le matériau bio-compatible auto expansible susmentionné.

Le treillis porteur cylindrique 20 est ici recouvert d'une enveloppe latérale étanche 21 destinée à être plaquée sur la paroi aortique pour éviter un contournement de la circulation sanguine.

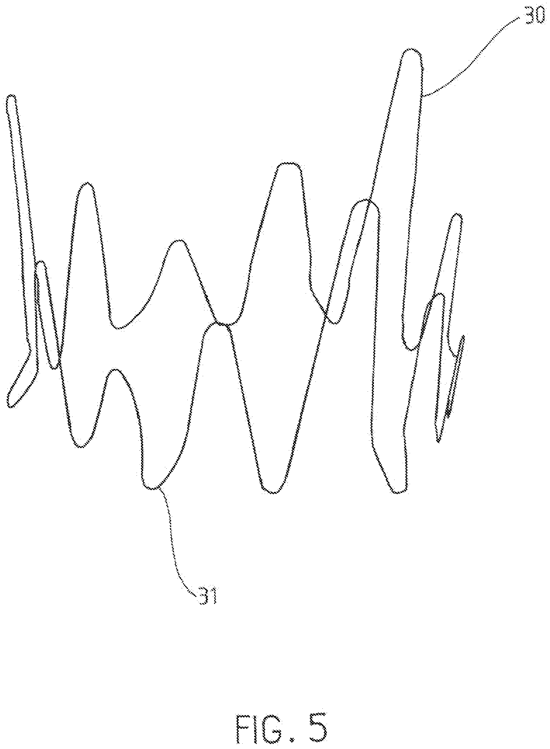

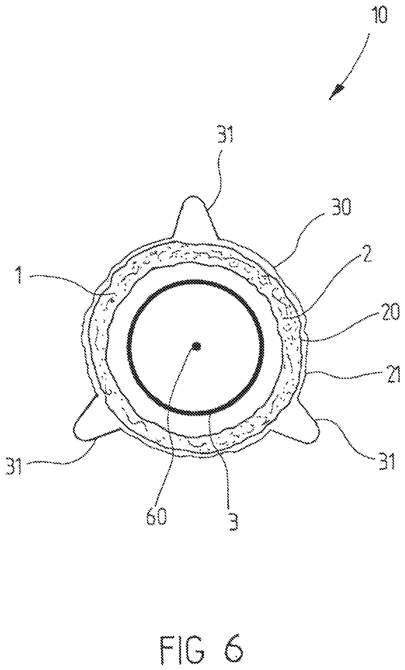

La figure 5 représente en perspective les éléments palpeurs 30, 31. La figure 6 est une vue schématique, selon une direction axiale du dispositif 10, montrant les trois boucles 31 faisant saillie latéralement à l'extérieur du grillage tubulaire 20 qui les porte, alors que les valvules 2 de la valve à implanter sont fixées intérieurement au cylindre porteur 20.

En outre, si nécessaire, un ballonnet 3 gonflable, solidaire du cathéter 60, peut être ici placé à l'intérieur du cylindre porteur 20, pour être alimenté en liquide sous pression à travers un conduit du cathéter 60 afin de provoquer ou d'aider l'expansion radiale du cylindre porteur 20 jusqu'à la forme déployée voulue.

Comme les éléments palpeurs 30, 31 sont fabriqués en un matériau autoexpansible tel que le nitinol, ou un élément équivalent formant patte ou doigt de saillie élastique, le dispositif 10 est recouvert d'une gaine d'inhibition 42 pour maintenir les éléments palpeurs 30, 31 en une position reployée, les boucles 31 étant rabattues sur la couronne 30 et donc aussi sur le treillis 20. La gaine 42 se prolonge pour recouvrir tout le cathéter 60. Une deuxième gaine 41, sensiblement de même longueur et sans effet sur les éléments palpeurs 30, 31, est ici de même prévue pour maintenir le cylindre porteur 20 en position reployée, afin d'éviter un déploiement inopiné même en l'absence de gonflage du ballonnet 3. Les deux gaines 41, 42, sont montées concentriquement sur le cathéter 60. Les gaines 41 et 42 sont accessibles depuis l'extrémité du cathéter 60 opposée au dispositif 10. Les éléments 3, 41, 42 et 60 constituent un ensemble cathéter fonctionnel séparable du dispositif 10, pour le positionnement et la mise en service de ce dernier et de sa charge utile (2).

Les deux gaines 41, 42 inhibent le déploiement radial de la structure 20, 30, 31 jusqu'à ce que celle-ci atteigne la région de la valve native aortique 50 à remplacer fonctionnellement, et permettent ainsi l'introduction du dispositif 10 dans le système de circulation sanguine, telle qu'une artère incisée de diamètre réduit. Comme indiqué, le cathéter 60, avec le ballonnet 3, est solidarisé de façon amovible au dispositif d'implantation 10 afin de permettre une avance axiale du dispositif d'implantation 10 dans le système de circulation sanguine jusqu'à l'endroit de l'implantation, et le retrait de l'ensemble cathéter 3, 41, 42, 60.

Pour sa libération, le cathéter 60 comporte dans cet exemple, à l'extrémité fixée au cylindre porteur 20, une pince à effet ressort (non représentée), avec des crocs télécommandés, montés rotatifs radialement, de solidarisation au dispositif 10 et il comporte un fil métallique central coulissant de télécommande pour repousser axialement les branches ou crocs de la pince afin de les écarter radialement et ainsi libérer le cathéter 60 du dispositif 10 d'implantation selon le principe d'une pince à sucre.

Lorsque le treillis cylindrique 20 est déployé, la pression sur la paroi interne aortique est assurée par l'effet de la mémoire de forme qui assure ainsi la dilatation radiale de la prothèse 1. La valvule native défaillante 50 est aplatie en étant plaquée par le grillage tubulaire 20 contre la paroi interne de l'aorte, chacune des trois boucles 31 faisant saillie latéralement ayant préalablement été engagée dans une, particulière, des trois valvules natives 50 et étant de même plaquée pour confirmer son ancrage. Les valvules 50 sont ainsi pincées entre le treillis 20, 30 et les boucles respectives 31.

Le procédé d'implantation du dispositif 10 décrit ci-dessus, selon le mode préféré de mise en oeuvre, comporte les étapes suivantes. Après introduction du dispositif 10 d'implantation dans le système circulatoire, et après l'avoir poussé au moyen du cathéter 60 jusqu'à une position en amont de la position finale visée, précisément ici lorsque le dispositif 10 est arrivé dans l'aorte, et qu'un espace de plus grand diamètre lui est ainsi offert, l'étape suivante consiste à libérer les boucles latérales 31, initialement plaquées sur le treillis 20, 30 reployé. La libération des boucles 31 s'effectue par retrait de la gaine externe d'inhibition 42 (figure 2), c'est-à-dire recul, en maintenant la poussée sur le cathéter 60. L'avance du dispositif 10 se poursuivant, les boucles 31, faisant alors nettement saillie latéralement vers l'avant par rapport à la direction axiale d'avance, à l'opposé du cathéter 60, elles constituent une sorte de trépied et pénètrent simultanément dans les trois valvules natives 50 respectives, sensiblement identiques, constituant une rangée en anneau complet de poches jointives, s'étendant chacune sur 120 degrés, garnissant au total la totalité du pourtour de la paroi interne de l'aorte 51. Chaque valvule native 50 présente un fond arrondi.

Chaque saillie latérale 31, tournée vers l'avant, bute sur le fond de la valvule 50 native en regard, en général en un point quelconque à distance du point le plus "bas" de ce fond, c'est-à-dire le plus éloigné du cathéter 60. Il s'agit alors d'une butée partielle car l'avance axiale du dispositif 10 se poursuit par poussée du cathéter 60, la poussée axiale sur le dispositif 10 provoquant son glissement vers le point le plus bas. Le fond de la valvule 50 constitue ainsi une sorte de piste de guidage ou plan incliné (non orthogonal à l'axe (62) de l'aorte) qui, par réaction à la force d'avance axiale, engendre une force de réaction circonférentielle provoquant la rotation du dispositif 10 jusqu'à ce que la boucle de palpeur considérée 31 atteigne le point le plus bas, qui correspond à une butée totale (à plan tangent orthogonal à l'axe (62) de l'aorte 51), donc correspond à la position finale recherchée, axiale et angulaire, du dispositif 10.

Chaque saillie latérale 31, à extrémité arrondie, ici en boucle, pour pouvoir glisser sur le fond de la valvule 50, constitue ainsi, par coopération avec le fond arrondi des valvules natives 50 de profondeur variable de façon continue, un moyen d'entraînement en rotation des éléments palpeurs 30, 31 et donc aussi du treillis cylindrique 20, qui en est solidaire. Toutefois, si par hasard les saillies latérales 31 butaient sur une commissure des valvules natives 50, le dispositif 10 d'implantation pourrait être légèrement reculé et l'opérateur tordrait le cathéter 60 pour qu'il pivote angulairement afin de recommencer l'opération de positionnement et d'ancrage.

L'ensemble, éléments palpeurs 30, 31 et treillis cylindrique 20, étant ainsi positionné axialement et angulairement par rapport au relief particulier de l'aorte que constituent les valvules natives 50, il se trouve donc automatiquement positionné par rapport aux deux orifices coronariens (52) dont la position axiale et angulaire par rapport aux valvules 50 est déterminée et connue, la distance axiale valvules-coronaires dépendant évidemment de la taille du patient.

Dans le cas ici considéré dans lequel les trois valvules natives 50 forment un pourtour circulaire de la paroi aortique s'étendant sur 360 degrés, une seule saillie latérale 31 est suffisante pour le positionnement modulo 120 degrés et l'ancrage du treillis cylindrique 20. Comme évoqué plus haut, dans un cas général, il pourrait n'y avoir qu'un seul palpeur 30, 31 coopérant avec une rangée de cavités ou poches garnissant tout le pourtour de l'élément tubulaire, ou bien encore une seule poche ou cavité 50 n'occupant qu'un secteur du pourtour et une pluralité de palpeurs 30, 31 tout autour du dispositif 10 pour que l'un d'entre eux coopère avec la cavité.

On notera que, dans le présent exemple, on peut tolérer un positionnement modulo 120 degrés car les deux coronaires (52) présentent naturellement sensiblement cet angle. Si ce n'était pas le cas, il faudrait élargir latéralement deux ouvertures ou échancrures 22 prévues dans l'enveloppe 21 pour qu'elles soient positionnées en face des coronaires (52) (fig. 4 et position repérée sur la fig. 3), ou encore palper, par les palpeurs 31, les coronaires (52) elles-mêmes, qui constituent aussi des cavités de l'aorte 51, et non plus palper les valvules natives 50. Ce cas correspond à la variante exposée plus loin.

Le positionnement étant ainsi effectué, l'étape suivante, telle que visualisée à la figure 3, consiste à déployer le treillis cylindrique 20 portant intérieurement les valvules 2 par retrait de la gaine interne 41 de maintien, pour consolider l'ancrage et faire passer les valvules 2 à leur forme fonctionnelle. Pour la clarté du dessin, en particulier des saillies 31, le treillis 20 a été représenté à un diamètre relatif réduit, alors qu'en fait il correspond à celui de l'aorte 51, avec un léger supplément pour assurer la pression latérale voulue. De même, on a représenté deux saillies 31, alors qu'en fait elles sont écartées de 120 degrés, le plan de la figure 3 n'en coupant en réalité qu'une seule. Pour cette raison, il n'a été dessiné qu'une seule coronaire (52).

Les trois boucles 31 faisant saillie assurent toutefois par elles-mêmes un ancrage élémentaire dans le fond des poches que constituent les valvules natives 50 et elles garantissent ainsi une stabilité de position de la prothèse 1. Après quelques semaines, un tissu fibreux viendra recouvrir la prothèse 1, coopérant avec les saillies latérales 31 pour améliorer encore sa fixation.

On notera toutefois que, en position déployée des éléments palpeurs 31, il n'est pas nécessaire que les extrémités libres de ceux-ci soient en appui ferme sur la paroi de l'aorte 51. Il suffit que leur extension radiale soit suffisante pour qu'elles s'accrochent, au passage, aux valvules 50. De ce fait, lorsque le déploiement des éléments palpeurs 31 s'effectue, en amont de la position finale, la translation axiale ultérieure du dispositif 10, jusqu'à cette position, s'effectue sans frottement "dur", sous pression, de la part des boucles 31 sur la paroi de l'aorte 51. Celle-ci ne court ainsi aucun risque d'endommagement par éraflure ou perçage, les boucles 31 étant des palpeurs, qui suivent la paroi de l'aorte 51 pour détecter les valvules 50. Comme évoqué plus haut, des pattes ou languettes à bout arrondi peuvent aussi convenir.

Les boucles de palpeur 31 n'ont donc pas ici pour fonction principale d'ancrer de façon très ferme le dispositif 10 dans l'aorte 51, puisqu'elles ne visent pas à excercer une forte presson radiale d'ancrage. Comme indiqué plus haut, il ne s'agit que d'un ancrage élémentaire. C'est ensuite le déploiement radial du treillis 20 qui engendre, par la mémoire de forme, une pression radiale d'ancrage définitif qui plaque sous pression le treillis 20 contre la paroi de l'aorte 51 et bloque ainsi tout déplacement relatif, tel que recul du dispositif 10 qui serait dû au flux sanguin, de direction opposée à celle de l'introduction du dispositif 10. Les éléments palpeurs 31 sont alors fonctionnellement superflus. Ils contribuent toutefois au maintien en position par le pincement des valvules 2. Comme le treillis 20 présente une surface de contact avec l'aorte 51 relativement élevée, tout risque d'endommagement de celle-ci est exclu. Le matériau à mémoire de forme permet de déterminer précisément la pression radiale exercée sur l'aorte 51, le diamètre ainsi accru de celle-ci étant donc parfaitement défini, ce qui élimine tout risque de contrainte radiale excessive.

Le procédé de l'invention peut être mis en oeuvre de façon non chirurgicale et dans un but non thérapeutique, pour implanter le dispositif 10 (ou équivalent) à une position déterminée dans un élément tubulaire présentant une paroi comportant une cavité, le procédé comportant les étapes suivantes :
un utilisateur introduit le dispositif (10) par une extrémité ouverte de l'élément tubulaire,
l'utilisateur actionne les moyens d'entraînement (60) (cathéter, aimant externe ou autre) pour faire avancer le dispositif (10) jusqu'à une position en amont de la position déterminée,
il commande les moyens d'activation (42) des moyens palpeurs (30, 31) et, l'avance se poursuivant,
il arrête l'actionnement des moyens d'entraînement (60) lorsqu'il détecte un blocage de l'avance, traduisant le fait que les moyens palpeurs (30, 31) se sont positionnés dans la cavité.

Pour faciliter l'entraînement du dispositif 10, celui-ci peut être associé à une sorte de rostre 61 précurseur (fig. 1 à 3) formant guide, sous forme d'élément cylindrique de diamètre limité, solidaire du cathéter 60.

On notera que le dispositif d'implantation selon l'invention peut d'abord être implanté seul, sans implant ou charge utile, ce dernier pouvant être implanté dessus par la suite selon le même principe. En pareil cas, le dispositif de l'invention comporte des moyens de réception du deuxième support, à venir, de l'implant, agencés pour assurer le positionnement et l'ancrage du deuxième support à la fois axialement, par butée, et radialement, avec des moyens de détrompage angulaire tels que doigt ou cavité prévu pour coopérer avec un élément de forme complémentaire du deuxième support.

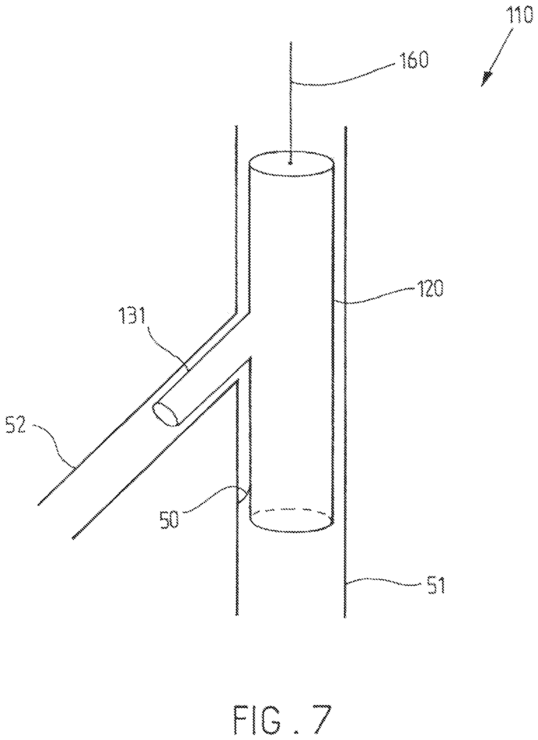

Dans la variante selon la figure 7, le dispositif d'implantation porte la référence 110 et comporte les éléments fonctionnels homologues de ceux du dispositif 10, avec la même référence précédée de la centaine 1, qui n'ont toutefois pas tous été représentés, dans un but de clarté. L'élément porteur cylindrique 120 est solidaire d'un élément palpeur 131 qui en fait saillie latéralement et qui présente une constitution de même genre que celle de l'élément porteur 120. Précisément, l'élément palpeur 131 se présente sous la forme d'un cylindre, reployé radialement au repos. Lorsque le dispositif 110 est poussé par le cathéter 160, vers le bas sur la figure 7, depuis une position en amont de celle représentée, il s'engage dans la coronaire 52 lorsque son extrémité libre est ainsi libérée du contact avec la paroi interne de l'aorte 51.

Le dispositif 110 constitue ainsi une sorte de fourche qui se bloque par butée dans la bifurcation entre l'aorte 51 et la coronaire 52. Lorsque la position de butée est atteinte, les deux éléments cylindriques 120, 131 sont déployés par deux ballonnets respectifs et forment une sorte de gant à deux doigts.

Ainsi, lors de la phase de mise en place, le palpeur 131 présente une forme reployée radialement, donc à diamètre réduit ne risquant pas d'obturer la coronaire 52. Ensuite, le palpeur 131 est déployé, par gonflage du ballonnet associé de téléactivation, et constitue un doublage, ou "enveloppe" interne, plaqué contre la paroi interne de la coronaire 52 selon le principe exposé précédemment pour le cylindre porteur 20.

On remarque que, comme les éléments 120 et 131 occupent chacun une branche particulière 51, 52, ils peuvent être considérés comme fonctionnellement homologues, avec éventuellement les deux fonctions principales. Chacun d'eux peut en effet être le porteur d'une charge utile (2) et peut aussi être considéré comme étant un palpeur, puisque l'aorte 51 peut être considérée (fonctionnellement dans le cadre de la présente invention) comme étant une cavité ou embranchement par rapport à la coronaire 52. Ainsi, les moyens palpeurs comportent un élément cylindrique 131 agencé pour passer d'une forme reployée à une forme déployée radialement, d'appui sur une paroi de la cavité, ici la coronaire 52, sous l'effet de moyens de téléactivation (ballonnet et cathéter 160).

Pour éviter les aléas de passage en position de couplage du palpeur 131 à la coronaire 52, dus à un décalage angulaire éventuel, qui nécessiterait plusieurs tentatives, il peut être prévu de passer préalablement un fil guide dans la coronaire 52 et la partie amont de l'aorte 51, le dispositif 110 étant enfilé dessus à travers le palpeur 131 qui est ainsi orienté angulairement vers la coronaire 52. Un autre fil guide peut de même guider le cylindre 120 dans l'aorte 51.

## Revendications

1. Dispositif pour implanter une prothèse de valve (2) à une position déterminée dans une aorte présentant une paroi comportant une cavité , le dispositif (10) étant agencé pour être relié de façon amovible à un cathéter (60) pour entraîner le dispositif dans l'aorte (51), le dispositif comprend :
- un élément porteur (20) apte à porter la prothèse de valve, et **caractérisé par** :
- un ensemble palpeur (30, 31, 131) solidaire à l'élément porteur, l'ensemble palpeur comprenant au moins un élément palpeur déformable et agencé pour passer d'une forme reployée à une forme déployée fonctionnelle sous le contrôle de moyens de téléactivation , l'ensemble palpeur étant apte à détecter la cavité et s'y positionner en référence à la position de la cavité.

2. Dispositif selon la revendication 1, dans lequel au moins un élément palpeur (30, 31) est à base d'un matériau à mémoire de forme.

3. Dispositif selon l'une des revendications 1 à 2, dans lequel l'ensemble palpeur (131) comporte un élément cylindrique agencé pour passer d'une forme reployée à une forme déployée radialement, d'appui sur une paroi de la cavité sous l'effet de moyens de téléactivation.

4. Dispositif selon l'une des revendications 1 à 2, dans lequel l'ensemble palpeur (30, 31) comporte une couronne, de forme globalement cylindrique, de boucles (31) de fil à rigidité limitée en direction radiale, l'une au moins des boucles (31) étant prévue pour faire saillie latéralement afin de constituer un élément palpeur.

5. Dispositif selon l'une des revendications 1 à 2, dans lequel l'ensemble palpeur (30, 31) comporter une pluralité d'éléments palpeur (31) régulièrement répartis angulairement et agencés pour, en forme déployée, s'étendre selon des directions respectives inclinées, à angle aigu, sur un axe longitudinal (62) d'entraînement en avant du dispositif vers la cavité.

6. Dispositif selon l'une des revendications 1 à 5, dans lequel l'élément porteur (20) est agencé pour, sous l'action de moyens de libération , passer d'une forme reployée à une forme déployée radialement, d'appui sur la paroi de l'aorte pour la mise en service de la prothèse de valve (2).

7. Système pour implanter une prothèse de valve (2) à une position déterminée dans une aorte , le système comprenant un dispositif selon l'une des revendications 1 à 6, les moyens de téléactivation (42) et le cathéter (60).

8. Système selon la revendication 7, dans lequel les moyens de téléactivation sont amovibles et comportent une gaine d'inhibition (42) pour maintenir un élément palpeur (30, 31) en position reployée, la gaine d'inhibition s'étendant au-delà de élément palpeur (30, 31) pour libérer l'élément palpeur par recul relatif de la gaine d'inhibition par rapport au cathéter (60).

9. Système selon la revendication 8 et comprenant un dispositif selon la revendication 6, dans lequel l'élément porteur (20) comporte un treillis de forme globalement cylindrique, pour porter la prothèse de valve, présentant une rigidité limitée en direction radiale, le système comprenant les moyens de libération qui comportent une gaine d'inhibition (41) pour maintenir le treillis en forme reployée, ladite gaine d'inhibition étant amovible et s'étendant axialement au-delà du treillis pour le libérer par recul relatif de la gaine (41) par rapport au cathéter (60), les gaines (41, 42) d'inhibition, pour maintenir en forme reployée respectivement l'élément palpeurs (30, 31) et le treillis, étant concentriques.

10. Système selon l'une des revendications 8 et 9, dans lequel l'élément porteur (20) est recouvert d'une enveloppe latérale (21) d'étanchéité, destinée à être plaquée sur la paroi de l'aorte par l'élément porteur et l'enveloppe (21) comporte au moins une ouverture ou échancrure (22) occupant une position angulaire déterminée par rapport à l'ensemble palpeur.

## Patentansprüche

1. Vorrichtung zum Implantieren einer Ventilprothese (2) an einer bestimmten Position in einer Aorta, die eine Wand aufweist, die einen Hohlraum umfasst, wobei die Vorrichtung (10) eingerichtet ist, um abnehmbar mit einem Katheter (60) verbunden zu sein, um die Vorrichtung in der Aorta (51) anzutreiben, wobei die Vorrichtung Folgendes umfasst:
- ein Tragelement (20), das geeignet ist, um die Ventilprothese zu tragen und **gekennzeichnet ist durch**:
- eine Fühlereinheit (30, 31, 131), die mit dem Tragelement verbunden ist, wobei die Fühlereinheit mindestens ein verformbares Fühlerelement umfasst, das eingerichtet ist, um von einer zurückgefalteten Position zu einer aufgefalteten Funktionsform unter der Steuerung von Fernbetätigungsmitteln überzugehen, wobei die Fühlereinheit geeignet ist, den Hohlraum zu erfassen und sich dort in Bezug zu der Position des Hohlraums zu positionieren.

2. Vorrichtung nach Anspruch 1, wobei das mindestens eine Fühlerelement (30, 31) auf der Basis eines Werkstoffs mit Formgedächtnis hergestellt ist.

3. Vorrichtung nach einem der Ansprüche 1 bis 2, wobei die Fühlereinheit (131) ein zylindrisches Element umfasst, das eingerichtet ist, um von einer zurückgefalteten Position auf eine radial aufgefaltete Position des Stützens auf einer Wand des Hohlraums unter der Einwirkung von Fernbetätigungsmitteln überzugehen.

4. Vorrichtung nach einem der Ansprüche 1 bis 2, wobei die Fühlereinheit (30, 31) einen Kranz mit insgesamt zylindrischer Form, Schleifen (31) aus Draht mit in radiale Richtung begrenzter Steifigkeit umfasst, wobei mindestens eine der Schleife (31) vorgesehen ist, um seitlich vorzustehen, um ein Fühlerelement zu bilden.

5. Vorrichtung nach einem der Ansprüche 1 bis 2, wobei die Fühlereinheit (30, 31) eine Vielzahl von Fühlerelementen (31) umfasst, die regelmäßig winkelig verteilt und eingerichtet ist, um sich in aufgefalteter Position entlang der jeweiligen schrägen Richtungen mit spitzem Winkel auf einer Längsachse (62) des Antreibens nach vorn von der Vorrichtung zu dem Hohlraum zu erstrecken.

6. Vorrichtung nach einem der Ansprüche 1 bis 5, wobei das Tragelement (20) eingerichtet ist, um unter der Einwirkung von Freigabemitteln von einer zurückgefalteten Form zu einer radial aufgefalteten Form zum Stützen auf der Wand der Aorta für die Inbetriebnahme der Ventilprothese (2) überzugehen.

7. System zum Implantieren einer Ventilprothese (2) an einer bestimmten Position in einer Aorta, wobei das System eine Vorrichtung nach einem der Ansprüche 1 bis 6, die Fernbetätigungsmittel (42) und den Katheter (60) umfasst.

8. System nach Anspruch 7, wobei die Fernbetätigungsmittel abnehmbar sind und eine Inhibitionshülle (42) zum Halten eines Fühlerelements (30, 31) in zurückgefalteter Position umfassen, wobei sich die Inhibitionshülle über das Fühlerelement (30, 31) erstreckt, um das Fühlerelement durch relatives Zurückbewegen der Inhibitionshülle in Bezug zu dem Katheter (60) freizugeben.

9. System nach Anspruch 8, und das eine Vorrichtung nach Anspruch 6 umfasst, wobei das Tragelement (20) ein Gitter mit im Allgemeinen zylindrischer Form umfasst, um die Ventilprothese zu tragen, das eine Steifigkeit aufweist, die in radiale Richtung begrenzt ist, wobei das System Freigabemittel umfasst, die eine Inhibitionshülle (41) zum Halten des Gitters in zurückgefalteter Position umfassen, wobei die Inhibitionshülle abnehmbar ist und sich axial über das Gitter hinaus erstreckt, um es durch relatives Zurückbewegen der Inhibitionshülle (41) in Bezug zu dem Katheter (60) freizugeben, wobei die Inhibitionshüllen (41, 42) konzentrisch sind, um das Fühlerelement (30, 31) und das Gitter jeweils in zurückgefalteter Form zu halten.

10. System nach einem der Ansprüche 8 und 9, wobei das Tragelement (20) mit einem seitlichen Abdichtmantel (21) abgedeckt ist, der dazu bestimmt ist, auf der Wand der Aorta von dem Tragelement angedrückt zu werden, und wobei der Mantel (21) mindestens eine Öffnung oder einen Ausschnitt (22) umfasst, die/der eine in Bezug zu der Fühlereinheit bestimmte Winkelposition belegt.

## Claims

1. Device for implanting a prosthetic valve (2) at a defined position in an aorta having a wall comprising a cavity, the device (10) being arranged to be removably connected to a catheter (60) to actuate the device in the aorta (51), the device comprises:
- a carrier element (20) suitable for carrying the prosthetic valve, and **characterised by**:
- a feeler assembly (30, 31, 131) secured to the carrier element, the feeler assembly comprising at least one deformable feeler element and arranged to switch from a retracted mode to a functional deployed mode under the control of remote activation means, the feeler assembly being suitable for detecting the cavity and positioning itself therein with reference to the position of the cavity.

2. Device according to claim 1, wherein at least one feeler element (30, 31) is based on a shape-memory material.

3. Device according to any one of claims 1 to 2, wherein the feeler assembly (131) comprises a cylindrical element arranged to switch from a retracted mode to a radially deployed mode, bearing on a wall of the cavity under the effect of remote activation means.

4. Device according to any one of claims 1 to 2, wherein the feeler assembly (30, 31) comprises a ring, having an overall cylindrical shape, of loops (31) of thread with limited rigidity in the radial direction, at least one of the loops (31) being adapted to protrude laterally in order to form a feeler element.

5. Device according to any one of claims 1 to 2, wherein the feeler assembly (30, 31) comprises a plurality of feeler elements (31) evenly angularly distributed and arranged to, in the deployed mode, extend along inclined respective directions, at an acute angle, along a longitudinal axis (62) for actuating the device forwards towards the cavity.

6. Device according to any one of claims 1 to 5, wherein the carrier element (20) is arranged, under the action of release means, to switch from a retracted mode to a radially deployed mode, bearing on the wall of the aorta for the activation of the prosthetic valve (2).

7. System for implanting a prosthetic valve (2) at a defined position in an aorta, the system comprising a device according to any one of claims 1 to 6, the remote activation means (42) and the catheter (60).

8. System according to claim 7, wherein the remote activation means are removable and comprise an inhibition sheath (42) for holding a feeler element (30, 31) in the retracted position, the inhibition sheath extending beyond the feeler element (30, 31) to release the feeler element by relative backward movement of the inhibition sheath with respect to the catheter (60).

9. System according to claim 8 and comprising a device according to claim 6, wherein the carrier element (20) comprises a mesh having an overall cylindrical shape, for carrying the prosthetic valve, having a limited rigidity in the radial direction, the system comprising release means comprising an inhibition sheath (41) for holding the mesh in the retracted mode, said inhibition sheath being removable and extending axially beyond the mesh in order to release same by relative backward movement of the sheath (41) with respect to the catheter (60), the inhibition sheaths (41, 42), for holding in the retracted mode the feeler elements (30, 31) and the mesh, respectively, being concentric.

10. System according to any one of claims 8 and 9, wherein the carrier element (20) is covered with a lateral sealing casing (21), intended to be placed on the wall of the aorta by the carrier element and the casing (21) comprises at least one opening or notch (22) occupying a defined angular position with respect to the feeler assembly.
